# EUROPEAN PATENT APPLICATION

(11) **EP 2 260 898 A1**
(43) Date of publication of application: **15.12.2010**
(21) Application number: 09162375.1
(22) Date of filing: 10.06.2009
(51) Int. Cl.: A61M 25/09

(54) **Guide wire and method for its use**

(71) Applicant: Schäfer, Ulrich, 20249 Hamburg (DE)
(72) Inventor: Schäfer, Ulrich, 20249 Hamburg (DE)
(74) Representative: HOFFMANN EITLE

(57) **Abstract**

The present disclosure pertains to a auide wire (1) for guiding intervention devices, the guide wire having a guide wire body (3) and a distal tip portion (10), wherein an expansion element (2) is present having, in an insertion configuration, a first diameter (D₁) which is substantially identical to the diameter (d) of the guide wire body(3) and, in an intervention configuration, a second diameter (D₂) which is substantially larger than the first diameter.

## Description

### Technical Field

The present invention pertains to a guide wire for guiding intervention devices into bodily lumens, in particular for guiding the insertion of intervention devices for the implantation of arterial stents and/or the implantation of artificial heart valves into bodily lumens. The present invention, furthermore, pertains to a method for using the guide wire.

### Technological Background

In the field of guide wires for guiding the insertion of intervention devices into bodily lumens of a patient, in particular for the insertion of intervention devices for the implantation of arterial stents and/or artificial heart valves into the blood vessels or heart of a patient, the stiff guide wires (typically 0.035") have, in order to reduce the risk of injuring the walls of the bodily lumen, a substantially blunt tip. This blunt tip is typically provided in the form of a spherical element that is situated at the distal tip portion of the guide wire, most frequently in the form of a semi-spherical end plug.

The blunt tip end of the conventional, extra stiff, guide wires is intended to reduce or avoid the risk of injuries of the blood vessels or heart muscle when advancing the guide wire into its intervention position. The blunt tip of the conventional guide wires is also intended to reduce or avoid the risk of injuries of the walls of the blood vessels or the heart muscle during the actual intervention.

Conventional guide wires with blunt tips are disclosed, for example, in EP 1 249 252 A2 and EP 1 388 350 A1.

### Summar of the Invention

It is an object of the present invention to further improve the safety aspects of a guide wire during the actual intervention and to improve the handling of the guide wire for the operator/ interventionalist, in particular in the field of cardiology, radiology and angiology.

These objectives are solved by a guide wire with the features of claim 1. Accordingly, a guide wire for guiding intervention devices is provided, the guide wire having a guide wire body and a distal tip portion. An expansion element is provided having, in an insertion configuration, a first diameter and, in an intervention configuration, a second diameter which is substantially larger than the first diameter.

The terms "distal" and "proximal" are used in this application in the sense that is usually assigned to them in connection with surgical instruments, namely the term "distil" indicating the end of a surgical instrument remote from the operator when the instrument is in use, and the term "proximal" indicating the end of the surgical instrument that is near to the operator when the surgical instrument is in use. In other words, the distal end is the end of the surgical instrument which is inserted first into a patient's body and which typically is the end for manipulating the respective interventional side, and the proximal end is in the hands of the operator for manipulating the device.

The term "diameter" is intended to mean the actual outer dimensions of the guide wire and of the expansion element when measured in a plane perpendicular to the longitudinal axis of the guide wire body. Accordingly, the diameter of the guide wire as such is typically measured, as it is customary in medical devices, in inch ('') [=2,54 cm], or according to the French catheter scale in the unit French (F).

The term "guide wire body" is intended to refer to the remaining guide wire excluding the expansion element.

Accordingly, the diameter of the expansion element in the intervention configuration, namely in the expanded state, is measured in a plane perpendicular to the longitudinal axis of the guide wire. A larger diameter of the distal tip portion in the intervention configuration than in the insertion configuration of the guide wire is given as soon as the outer dimensions of the expansion element extend beyond the diameter defined by the guide wire body.

Even in a situation in which only two expansion element wires are used which are, in the intervention configuration shaped like a ring, the outer dimensions of the two expansion element wires are intended to define the diameter of the expansion element in this case, even though it appears to be just a distance.

The provision of the expansion element at the distal tip portion of the guide wire in the intervention configuration in which the expansion element is expanded such as to increase the outer diameter of the distal tip portion substantially over the diameter of the guide wire body has the effect that the risk of an injury of the walls of a bodily lumen, in particular the walls of a blood vessel or the walls of the heart muscle, is greatly reduced.

Because during the intervention operation the guide wire might be moving within the bodily lumen due to friction between the intervention device and the outer surface of the guide wire, the provision of the expansion element in the intervention configuration reduces the risk of any injury of the bodily lumen during these movements. Furthermore, the distal tip portion of the guide wire might be moving relative to the walls of the bodily lumen due to the movement of the bodily lumen. In particular, when using the guide wire for guiding an intervention device into a cardiac chamber during the implantation of an artificial heart valve, the distal tip portion of the guide wire might be moving relative to the wall of the heart muscle because the heart muscle contracts during the cardiac cycle. The provision of the expansion element in the intervention configuration reduces the risk of punctuations due to the relative movements of the distal tip end and the heart muscle. The expansion element may also provide stability to support the exact navigation of medical over-the-wire devices. The expansion element particularly might provide additional support under circumstances where an increased resistance and/or friction during the intervention occurs.

Accordingly, after having transformed the expansion element into the intervention configuration, the operating operator does not have to concentrate on the actual position of the distal tip end of the guide wire anymore because it is safe to remain unattended for a while, but can fully concentrate on the use, placement and application of the intervention device during the operation. This is even more so the case as the distal tip end of the guide wire might be fixed or immobilized in its intervention configuration within the respective bodily lumen, for example by jamming it by means of the expansion element.

Furthermore, the guide wire with the expansion element in its intervention configuration cannot move around freely and uncontrolled within the cardiac chambers during the cardiac cycle but the movement of the distal tip portion of the guide wire is substantially reduced.

The expansion element can be provided in the form of a wire mesh which can be expanded. In particular, the expansion element can be provided by means of at least two expansion element wires which are situated at the distal tip portion of the guide wire and which are situated in parallel to one another in the insertion configuration and which are substantially bent in the intervention configuration. By the provision of a wire mesh or the simple provision of two expansion element wires, a reliable guide wire can be provided, in which the outer diameter of the guide wire remains substantially unchanged, at least in the insertion configuration of the expansion member, at the position of the distal tip portion.

In order to be in a position to transform the expansion element from the insertion configuration to the intervention configuration, the guide wire may include a core wire and an actuating wire, wherein the actuating wire is movable with respect to the core wire and wherein the actuating wire is connected to the expansion element such that its actuation expands the expansion element from the insertion configuration to the intervention configuration and retracts it back to the insertion configuration.

The core wire and the actuation wire may be provided either in the form of a Bowden cable in which the two wires are situated substantially concentrically with respect to one another, or in an arrangement in which the two wires are provided in parallel to one another, preferably covered by a sheath.

A guide wire provided in this manner allows for a mechanically reliable setup of the guide wire which ensures a secure operation of the expansion element. The provision of an actuating wire functions in combination with any bendable material for the expansion element, preferably a stainless-steel.

In an alternative, the guide wire includes a core wire and a retractable sheath/guide catheter, wherein the retractable sheath is situated around the expansion element in the insertion configuration and is retracted from the expansion element in the intervention configuration. By retracting the sheath from the expansion element, the expansion element is set free to expand into the intervention configuration. Preferably, the expansion element is made self-expanding to expand into the intervention configuration, and is preferably made from a shape-memory material, preferably a Nitinol or hyperelastic metal, a hyperelastic metal, a spring metal, or any pre-tensioned material which automatically self-expands after the sheath is retracted from the expansion element.

In another embodiment, the expansion element is provided in the form of an inflatable element, which is situated at the distal tip portion of the guide wire.

The expansion element may also be provided in the form of an inflatable element which is situated at the distal tip portion of the guide wire.

The guide wire is particularly suited for guiding intervention devices to their intervention position through a bodily lumen of a patient, in particular for guiding the insertion of an intervention device for the implantation of a stent into a blood vessel or the implantation of an artificial valve into the heart.

The guide wire is suitable for interventions for occluding left atrial appendages by means of conventional plugs or umbrellas (e.g. Amplatzer, Watchman, Plato-Devices). In this specific case the first diameter of the expansion element is preferably identical to the guide wire body (remaining guide wire).

The objective set out above is also solved by a method for using a guide wire according to claim 13. In particular, in a first step, the guide wire in its insertion configuration is inserted into a bodily lumen of a patient, in a second step, the expansion element is transformed into the intervention configuration at the position where the distal tip portion of the guide wire is intended to be situated during the intervention, as the third step, an intervention device is guided to its intervention position by means of the guide wire, as the fourth step, the intervention device is retracted from the bodily lumen of the patient after the intervention has been carried out, and as the last step the expansion element is brought back into the insertion configuration and the guide wire is retracted from the bodily lumen of the patient.

### Brief Description of the Figures

The invention will be discussed in more detail below, with reference to the Figures, in which:
- Figures 1a and 1b: are schematic perspective views of a guide wire according to an embodiment in which an expansion element is shielded by means of a retractable sheath and can be expanded by retracting the sheath;
- Figures 2 to 4: are different stages of the expansion process of the guide wire shown in Figures 1a and 1b;
- Figures 5a and 5b: show a guide wire in which the expansion element can be expanded by using an actuating wire which can be moved relative to a core wire of the guide wire;
- Figures 6 to 9: show different expansion states of the guide wire according to Figure 5.

### Detailed Description of the Figures

In the following, different embodiments of the present invention will be described by reference to the Figures. Identical or similar features will be denoted by the same reference numerals and repeated description thereof may be omitted, in order to reduce redundancies.

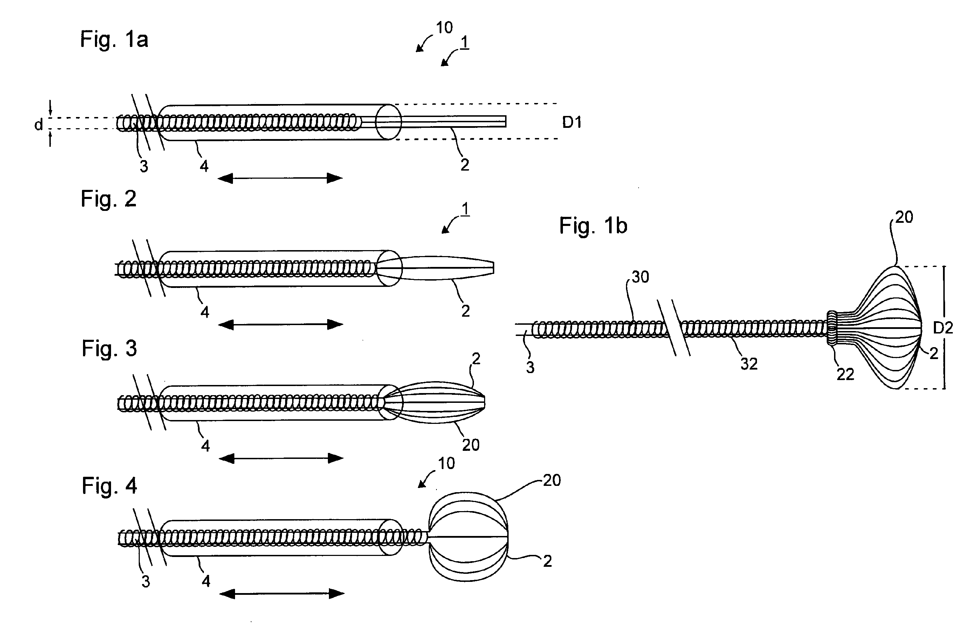

Figures 1a and 1b show a guide wire 1 in an insertion configuration (Fig. 1a) and an intervention configuration (Fig. 1b). The guide wire has, at its distal tip end 10, an expansion element 2 which will be described in more detail in the following. The insertion configuration is used when inserting the guide wire 1 into a bodily lumen of a patient until the distal tip end 10 reaches its final position, and the intervention configuration is used during an intervention device is guided by means of the guide wire into the bodily lumen of the patient.

In the insertion configuration, the expansion element 2 may have substantially the same outer diameter D₁ as the diameter d of the guide wire body 3. The term "guide wire body" is intended to refer to the remaining guide wire excluding the expansion element. However, D₁ may also have a larger diameter than the diameter d of the guide wire body 3. The guide wire body 3 has, as a typical diameter d, a dimension of 0.035" (approx. 0.9 mm).

In the intervention configuration, the expansion element 2 is shown in an expanded state having a diameter D₂ which is substantially larger than the diameter d of the guide wire body 3. D₂ is also substantially larger than the diameter D₁ of the expansion element 2 in the insertion configuration. D₂ may be preferably in a range of 5-40mm, preferably between 10-35mm, more preferably between 20-30mm, most preferably 25mm.

The guide wire 1 comprises the guide wire body 3, which typically comprises a core wire 30 and a coil wire 32, preferably in the form as a typical setup for a conventional extra stiff guide wire. The outer diameter d of the guide wire 3 is defined by the outside of the coil winding 32 which is typically coated with PTFE or Heparin.

The main guide wire body 3 is used for guiding an intervention device to the desired location within a bodily lumen. In particular, intervention devices for the implantation of stents or artificial valves can be guided along the guide wire easily towards the desired intervention location.

The distal tip portion 10 of the guide wire 1 comprises expansion element 2. In the embodiment shown in Figures 1a and 1b, the expansion element 2 comprises several expansion element wires 20 which are formed from a shape-memory material, such as Nitinol, or from a spring-steel material which is biased towards the form of the expansion element 2 shown in Figure 1b. The shape-memory material or the biased material is in particular shaped such that the expansion element 2 recaptures the shape shown in Figure 1b and such that the outer diameter D₂ of the expanded expansion element 20 is substantially larger than the diameter d of the guide wire body 3.

The expansion element wires 20 are attached to the guide wire body, as can be seen in Figure 1b, by means of soldering at position 22, or by any other suitable means of attachment of the expansion element wires 20 to the guide wire body 3.

In order to be in a position to insert the guide wire according to Figures 1a and 1b into a bodily lumen and in order to restrain the expansion element 2 in the insertion configuration, namely the configuration shown in Figure 1a, a retractable sheath 4 is present. The retractable sheath 4 is situated around the expansion element 3 in Figure 1a in order to keep the expansion element 2 in its unexpanded configuration during the insertion of the guide wire 1.

As soon as the guide wire 1 is fully inserted into a bodily lumen of a patient and the distal tip portion 10 of the guide wire 1 has reached its intervention position, sheath 4 is retracted with respect to the expansion element 3 to set it free, such that the expansion element wires 20 can expand due to their bias or shape memory.

As will become apparent, the configuration as shown in Figure 1b is advantageous when it comes to a secure and safe placement of the distal tip portion 10 inside a bodily lumen of a patient, in particular when the bodily lumen is a cardiac chamber which moves quite heavily. It becomes immediately apparent that, due to the substantially expanded diameter D₂ of the expansion element 2, the risk of a punctuation of a wall of the bodily lumen is greatly reduced compared to the situation in which the guide wire 1 only has diameter d of the guide wire body 3.

Figures 2 to 4 show different steps during the retraction process of the sheath 4 with respect to the guide wire body 3, and in particular with respect to the expansion element 2. The retraction of the sheath 4 serves to set the expansion element 2 at the distal tip end 10 of the guide wire 1 free, in order to allow for an expansion of the expansion element wires 20 by means of the material used.

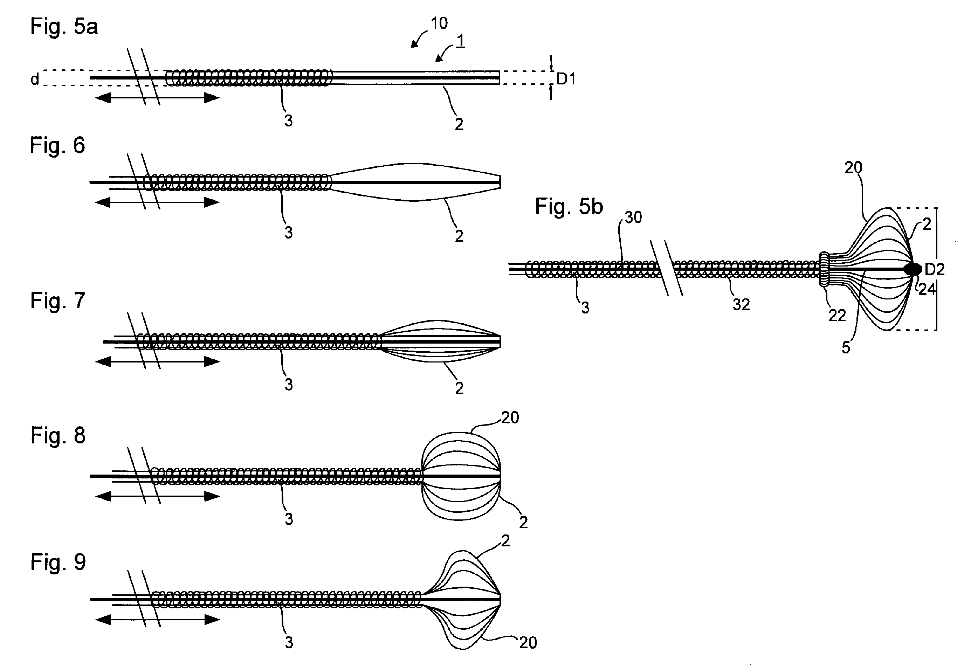

The guide wire 1 shown in Figure 5a in the insertion configuration and in Figure 5b in the intervention configuration likewise comprises, at its distal tip portion 10, an expansion element 2 which provided in such a form that it has a first outer diameter D₁ in the insertion configuration.

In the intervention configuration of the guide wire 1 shown in Figure 5b, the expansion member 2 has an outer diameter D₂ which is substantially larger than the first diameter D₁ of the expansion element 2 in the insertion configuration. The expansion element wires 20 of the expansion element 2 are provided, in this embodiment, in the form of a flexible material, but are not provided with any shape memory characteristics or any biasing of the material towards a bent configuration.

Accordingly, in order to bring the expansion element 2 from the insertion configuration, i.e. the unexpanded state, into the intervention configuration, i.e. the expanded state, the guide wire 1 further includes an actuating wire 5 which is carried in the guide wire body 3 and which can be moved relative to both, the core wire 30 and the coil wire 32. At its distal end, the actuating wire 5 is connected to the distal tip end 24 of the expansion element wires 20 such that, when the actuating wire 5 is retracted, i.e. moved in a proximal direction, the distal tip end 24 is likewise retracted. This retraction forces the expansion element wires 20 to expand in the form as shown in Figure 5b. of course, the arrangement can also be made such that the core wire is retracted proximally. Important for this manner of actuation is that the core wire and the actuation wire are moved relatively to one another in order to effect the expansion of the expansion member.

Furthermore, Figures 6 to 9 show different phases of the expansion of expansion element 2 by means of moving the actuating wire 5 relative to the guide wire body 3.

Nevertheless, in order to fully close the distal tip portion 10 of the guide wire 1 during insertion and during retraction, it is contemplated to provide the guide wire 1 shown in Figures 5 to 9 with a retractable sheath, preferably in the form of a regular catheter, in order to shield the guide wire.

In order to remove the guide wire from a bodily lumen of the patient again, the expansion element 2 is brought back into the insertion configuration, namely the configuration in which the outer diameter D₁ of the expansion element 2 is substantially identical to the outer diameter d of the guide wire core 3.

In order to bring the expansion element 3 back into the insertion configuration, either the retractable sheath 4 of Figures 1 to 4 can be pushed over the expansion element again, substantially reversing the steps shown in Figures 2 to 4, or the actuating wire 5 can be pushed in the distal direction, substantially reversing the steps shown in Figures 6 to 9.

## Claims

1. Guide wire (1) for guiding intervention devices, the guide wire having a guide wire body (3) and a distal tip portion (10),
**characterized by**
an expansion element (2) having, in an insertion configuration, a first diameter (D₁) and, in an intervention configuration, a second diameter (D₂) which is substantially larger than the first diameter.

2. Guide wire according to claim 1, wherein the expansion element is provided in the form of a wire mesh.

3. Guide wire according to claim 1 or 2, wherein the expansion element is provided by means of at least two expansion element wires (20) which are situated in parallel in the insertion configuration and which are substantially bent in the intervention configuration.

4. Guide wire according to any one of the preceding claims, wherein the guide wire includes a core wire (30) and an actuating wire (5), wherein the actuating wire is movable relative to the core wire and wherein the actuating wire is connected to the expansion element such that its actuation transforms the expansion element from the insertion configuration into the intervention configuration.

5. Guide wire according to any one of the preceding claims, wherein the guide wire includes a core wire (30) and a retractable sheath (4), wherein the retractable sheath covers the expansion element in the insertion configuration and is retracted from the expansion element in the intervention configuration.

6. Guide wire according to claim 5, wherein the retractable sheath is provided in the form of a conventional catheter.

7. Guide wire according to any one of the preceding claims, wherein the expansion element self-expands into the intervention configuration.

8. Guide wire according to any one of the preceding claims, wherein the expansion element is made from a shape-memory material, preferably Nitinol, a hyperelastic metal, a spring metal, or any suitable material which biases the expansion element into the intervention configuration.

9. Guide wire according to any one of the preceding claims, wherein the expansion element is provided in the form of an inflatable element.

10. Guide wire according to any one of the preceding claims, wherein the guide wire is intended for guiding intervention devices to their intervention position through a bodily lumen of a patient, in particular for guiding the insertion of an intervention device for the implantation of a stent into a blood vessel or the implantation of an artificial valve into the heart.

11. Guide wire according to any one of the preceding claims, wherein the first diameter (D₁) is substantially identical to the diameter (d) of the guide wire body(3).

12. Guide wire according to any one of the preceding claims, wherein the guide wire body has a diameter of 0.035 Inch, preferably in the form of an extra stiff or super stiff guide wire.

13. Method for using a guide wire according to any one of the preceding claims, wherein, in a first step, the guide wire is, in its insertion configuration, inserted into a bodily lumen of a patient, in a second step, the expansion element is transformed into the intervention configuration at the position where the distal tip portion of the guide wire is intended to be situated during the intervention, as the third step, an intervention device is guided to its intervention position by means of the guide wire, as the fourth step, the intervention device is retracted from the bodily lumen of the patient after the intervention has been carried out, and as the last step the expansion element is brought back into the insertion configuration and the guide wire is retracted from the bodily lumen of the patient.
